# EUROPEAN PATENT SPECIFICATION

(11) **EP 2 776 549 B1**
(45) Date of publication and mention of the grant of the patent: **10.01.2018**
(21) Application number: 12805771.8
(22) Date of filing: 12.11.2012
(51) Int. Cl.: C12C 5/00, C12C 11/00, C12G 1/02, C12G 3/02, A61K 8/97, A61Q 5/00, A61Q 5/02, A61Q 19/00, C12C 7/28

(54) **IMPROVED PROCESS FOR THE MANUFACTURE OF ALCOHOLIC BEVERAGES**
VERBESSERTES VERFAHREN ZUR HERSTELLUNG VON ALKOHOLISCHEN GETRÄNKEN
PROCÉDÉ AMÉLIORÉ POUR LA FABRICATION DE BOISSONS ALCOOLIQUES

(30) Priority: 11.11.2011 ZA 201108289
(43) Date of publication of application: 17.09.2014
(73) Proprietor: RED DAWN IP HOLDINGS (PTY) LTD., Stellenbosch 7613 (ZA)
(72) Inventor: STRYDOM, Trevor, 7600 Stellenbosch, Western Cape Province (ZA); VAN NIEKERK, Michael, 7600 Stellenbosch Western Cape Province (ZA); GHAI, Oliver, 1000 CC Amsterdam (NL)
(74) Representative: Titmus, Craig Edward
(86) International application number: PCT/IB2012/056352
(87) International publication number: WO 2013/068999

(56) References cited:
- EP-A2- 2 735 304
- WO-A1-2007/057310
- DE-U1- 29 916 515
- FR-A1- 2 879 446
- KR-A- 20040 039 207
- KR-A- 20110 091 606
- US-A- 2 692 199
- US-A- 3 998 761
- DATABASE WPI Week 200969 Thomson Scientific, London, GB; AN 2009-P02571 XP002692879, & CN 101 531 957 A (XIE K) 16 September 2009 (2009-09-16)
- MARNEWICK J L ET AL: "Effects of rooibos (Aspalathus linearis) on oxidative stress and biochemical parameters in adults at risk for cardiovascular disease", JOURNAL OF ETHNOPHARMACOLOGY, ELSEVIER SCIENTIFIC PUBLISHERS LTD, IE, vol. 133, no. 1, 7 January 2011 (2011-01-07), pages 46-52, XP027575269, ISSN: 0378-8741 [retrieved on 2010-12-24]
- JOUBERT E ET AL: "South African herbal teas: Aspalathus linearis, Cyclopia spp. and Athrixia phylicoides-A review", JOURNAL OF ETHNOPHARMACOLOGY, ELSEVIER IRELAND LTD, IE, vol. 119, no. 3, 1 October 2008 (2008-10-01), pages 376-412, XP009167003, ISSN: 0378-8741
- , 29 June 2011 (2011-06-29), Retrieved from the Internet: URL:homebru.net/2011/06/29/rooibos-beer--- the-taste-of-africa/ [retrieved on 2016-05-18]
- , 18 July 2009 (2009-07-18), Retrieved from the Internet: URL:www.ratebeer.com/beer/brasseurs-de-mon treal-rooibos-lafricaine/104728/1/4/ [retrieved on 2016-05-18]

## Description

### FIELD OF THE INVENTION

This invention relates to an improved process for the manufacture of an alcoholic beverage, and products produced by such process. More specifically, but not exclusively, the invention relates to an improved process for making a red wine product using selected grape cultivars, a beer product and a cider product.

### BACKGROUND TO THE INVENTION

***Wine*:** Processes for the manufacture of various alcoholic beverages, are known in the art, such as the process of making red wine from red wine grape cultivars and white wine from white wine grape cultivars. The red wine making process generally includes the steps of harvesting selected grapes from a vineyard, destemming the grapes if desired (this step is optional, especially in the case of manufacturing red wine) and proceeding to crush the grapes ("the crushing step") to form a so-called must, consisting of pulp, skin and seed.

Primary fermentation then follows by adding specialized cultured yeast to produce more predictable results in the final product. During primary fermentation, the yeast converts natural sugars in the must into alcohol and carbon dioxide, and tannins are extracted from the must, most notably the grape skin and seeds. After the step of primary fermentation is completed, the must is pressed ("the pressing step") in order to harvest additional juice (known as "pressed juice") in addition to the free-run juice already liberated during the crushing step. The pressing step also serves to separate the grape skin and other solid, residual matter from the liquid.

After pressing, the pressed and/or free-run juice combination is transferred to tanks or barrels where malolactic fermentation (or a secondary fermentation step) takes place. During malolactic fermentation, specifically selected strains of bacteria are added (which can also occur naturally) to convert malic acid to lactic acid, the latter being a milder acid. Additional tannins may be extracted from the wood in oak barrels or from oak inserts in instances where oak barrels are not used as the primary source for the extraction of oak tannins and oak flavours. Once malolactic fermentation is completed, the aforementioned juice combination (hereinafter referred to as *"wine"* or "a *wine product"*) will be racked off by-products in the form of sediment / lees created during the process of manufacture.

Completion of the secondary fermentation step permits wine to be left to age and mature over time, usually six to eighteen months for red wines.

Generally, an artificial preservative is added to the wine product, as the quality of a wine is dependant, *inter alia,* on its oxidative status. This artificial preservative takes the form of a sulphur source (usually sulphites, for example sulphur dioxide). The purpose of the sulphur source is twofold. Firstly, sulphur dioxide acts as an anti-microbial agent. Secondly, the sulphur source acts as an antioxidant to protect the wine from oxidation which would be detrimental to the wine product.

Excess levels of oxidation may lead to a decrease in the varietal character of the wine, with further oxidation leading to the formation of unwanted compounds such as acetaldehyde, sotolon, methional and phenylacetaldehyde, with associated flavours of sherry and green apple. Oxidation may also lead to unwanted color changes in the wine and the growth of spoilage organisms such as *Brettanomyces.* The antioxidant properties of the sulphur source thus ensure a longer shelf life for the wine product, once in a saleable condition. Sulphur may be added at any one of three stages, namely, during the crushing step, at the commencement of secondary fermentation, or as a penultimate step to completion of the process of manufacture. The addition of when sulphur is added can vary in the red and white wine making process.

Lastly, once aging and maturation have completed, the wine product can undergo further filtration, and may thereafter be bottled, and labelled. The wine product is at this stage ripe for delivery to the consumer market.

There may be certain disadvantages associated with the known process of manufacture detailed above, in particular with respect to the addition of sulphur to a wine product. For example, the addition of sulphur substances in the wine making process may have a detrimental effect on certain types of consumers. Some consumers, such as asthmatics, may have sensitivity to the presence of sulphur compounds present in wine and other products. These persons may as a result of consuming sulphur-containing wine products experience more intense hangover-simulating symptoms. Anaphylaxis of some degree as a result of the presence of sulphur preservatives remains a distinct possibility, which may cause such customers to avoid purchasing these products altogether.

Additionally, sulphur may also impart an unpleasant colour and taste to a wine which may be detectable by an experienced taster, making the product an unattractive one to purchase.

In spite of the side effects and negative connotation associated with sulphur-based preservatives in the context of wine products, the possibility of utilising an alternative to sulphur additives to negate or to reduce the level of artificial preservatives in these products has not been successfully explored and implemented in the industry to date.

Further, the quality of a wine is assessed on the basis of colour, aroma, taste and mouthfeel. These factors, and especially taste and mouthfeel may be affected by various polyphenols present and/or deposited in wine, which includes flavonoids. Flavonoids are polyphenolic compounds, which impart characteristic taste, colour and mouthfeel to a wine. Winemakers and consumers alike, aim to continually improve upon and discover new and/or improved combinations of tastes in wines.

***Beer*.** Processes for the manufacture of various other alcoholic beverages, such as the process of making beer, are known in the art. Beer is a well-known beverage that is widely consumed. It is produced in a process whereby starch is converted into sugar, which is in turn subjected to fermentation to produce alcohol. In the main the flavour and bitterness is derived from hops as an ingredient, which may also impart some natural preservative characteristics to the beer product.

Generally, the commercial production of beer is subjected to certain regulations and rules, the most well-known of which is the *Reinheitsgebot* (or Purity Law), dating back to the 16^{th} century and which is still used today. Under the Reinheidsgebot, the only ingredients that are allowed in the manufacture of beer are water, hops and barley-malt.

A handful of commercial brewers represent market leaders in a tightly contested market. In order to remain competitive, brewers are constantly tweaking and attempting improvements to their products within the strict confines of the purity law. Such modifications are of course limited. Focus is also being poised towards gaining a larger presence in emerging markets, while established markets may react positively to innovative marketing of current products. There is also competition from so-called micro-breweries offering alternative varieties in the flavour of beer products in order to stand out from the mass of similar beer products currently on offer.
***Cider:*** To begin the cider making process fruit (normally apples or pears) must be harvested, sorted, and washed. The cider making process typically involves three stages including crushing the fruit, pressing out juice, and allowing it to ferment. Cider consumption climbed from 2005 by 72 percent to about 440,000 hectolitres in 2006 in the United States of America. It is a known fact that several high-profile industry players have entered the market with malt-based drinks given an apple flavour "that they are trying to pass off as authentic ciders". These "pseudo ciders" are generally cheaper to produce than the real 'thing', allowing manufacturers to undercut genuine, authentic cider brands on price. In the case of a genuine cider, the taste is derived from the fermenting real apple juice rather than flavouring other alcoholic drinks with apple flavours. As a category brand, ciders continue to outstrip average growth in the liquor sector. The invention allows for the opportunity to produce a 'new' cider using conventional methods, but eradicating or reducing the levels of known preservatives, while at the same time making a cider with unique flavours, aroma's and mouthfeel.

KR 2004 0039207 discloses fermented wine produced from a blend of roasted black bean powder and boiled rice that is subjected to fermentation.

https://homebru.net/2011/06/29/rooibos-beer---the-taste-of-africa/ and https://www.ratebeer.com/beer/brasseurs-de-montreal-rooiboslafricaine/104728/1/4/ both relate to Rooibos-flavoured beer.

### OBJECT OF THE INVENTION

It is therefore a first object of the invention in regard to wine is either provide an improved process for the manufacture of an alcoholic beverage, more particularly but not exclusively to a low sulphur red wine (or white wine), with which the aforesaid disadvantages can be overcome or at least minimised or which will provide a useful alternative to existing methods or to provide a suitable alternative to oak wood and oak wood extracts in regard to the wine making process or to provide both improvements in both the making of red and white wine or in one of the wines.

It is a second object of the invention to provide a modified beer manufacturing process and alternative product to known processes and products.

It is a third object of the invention to provide a modified cider manufacturing process and alternative product to known processes and products.

It is a further object of the invention to provide improved alcoholic beverages having unique colours, aromas, flavours and mouth feel.

### SUMMARY OF THE INVENTION

In the context of this specification, the term "low-sulphur" as it applies to a wine product, or a beer product or a cider product to be interpreted as either containing no sulphur, or the sulphur concentration present in the aforementioned product is insufficient to cause a symptomatic reaction in a consumer. In particular, the concentration may be less than 10ppm.

The invention is defined according to the claims.

According to a first aspect of the invention, there is provided an improved process for the manufacture of an alcoholic beverage, including the steps of providing a sugar source, subjecting the sugar source in the presence of plant material, to at least one instance of fermentation, said plant material selected from the species *Aspalathus linearis* (rooibos), *Cyclopia* (honeybush), or a combination of these, or plant material of *Athrixia phylicoides* (Bush Tea), wherein said fermentation process potentiates extraction of polyphenols from the plant material, and wherein said polyphenols have preservative and unique flavour- and aroma- imparting properties.

The extractable compounds may also include amylalcohols and other compounds having preservative and unique flavour-imparting properties. The sugar source may be any one or more of a starch material, grape cultivars, apple cultivars, or pear cultivars. The alcoholic beverage may be a wine, beer product or a hard cider product.

According to a first embodiment of the invention there is provided an improved process for the manufacture of a low-sulphur alcoholic wine, including the steps of:
- providing the sugar source and deriving a must from the sugar source;
- subjecting the must to primary fermentation; and;
- applying known clarification, stabilisation, fining, and filtration methods to the must;
- the process of manufacture including the further step of simultaneously adding plant material selected from the species *Aspalathus linearis* (rooibos), *Cyclopia* (honeybush), or a combination of these, or plant material of *Athrixia phylicoides* (Bush Tea) during the step of primary fermentation, thereby potentiating extraction of polyphenols from the plant material, useful in imparting a unique flavour and aroma to the alcoholic beverage.

The step of primary fermentation may increase the efficacy of polyphenol extraction and may occur over a temperature range of between 15 degrees Centigrade and 90 degrees Centigrade. Preferably, the step of primary fermentation may occur over a temperature range of between 20 degrees Centigrade and 60 degrees Centigrade. Most preferably, the step of primary fermentation may occur over a temperature range of between 20 and 30 degrees Centigrade.

There may be further provided, according to the invention, that the process of manufacture of low sulphur wine may include a further and/or alternative step of optionally adding plant material selected from the species *Aspalathus linearis* (rooibos), *Cyclopia* (honeybush), or a combination of these, or plant material of *Athrixia phylicoides* (Bush Tea) during a secondary fermentation step. The secondary fermentation step may precede the step of applying known methods of clarification, stabilisation, fining, and filtration to the must, in order to produce a wine product.

The invention further provides that the plant material selected from the species *Aspalathus linearis* (rooibos), *Cyclopia* (honeybush), or a combination of these, or plant material of *Athrixia phylicoides* (Bush Tea) may include parts of the plant, including the roots, stems, leaves, branches, seeds, flowers, fruit and bark, or a combination of these. The plant material may further be in its natural form or may be in a processed form.

The invention further provides that the addition of the above mentioned material selected from the species *Aspalathus linearis* (rooibos), *Cyclopia* (honeybush), or a combination of these, or plant material of *Athrixia phylicoides* (Bush Tea) or components thereof, may replace oak wood or oak wood extracts in the wine making process, or may complement oak wood or oak wood extracts in the wine making process.

*Aspalathus linearis* (rooibos) and *Cyclopia* (honeybush) species are members of the Fabaceae family. Athrixia phylicoides (Bush Tea) is a member of the Asteraceae family.

The grape cultivars may be selected from any suitable grape cultivar, and more specifically any one or more of Merlot, Shiraz (or Syrah), Pinotage, Cabernet Sauvignon, Chardonnay, Chenin Blanc, Sauvignon Blanc, Semillon (the last four are white wine cultivars) or a combination of the foregoing.

The invention may further provide that the polyphenols extracted from the *Aspalathus linearis* (rooibos), *Cyclopia* (honeybush), or a combination of these, or the *Athrixia phylicoides* (Bush Tea) may include compounds from either of the groups, flavonoids or non-flavonoids. The flavonoids may include quercetin, luteolin, orientin, iso-orientin, vitexin, iso-vitexin and aspalathin, the latter being unique to *Aspalathus linearis.* The polyphenols extracted function as a natural preservative in the wine product. Described is a modified process for the manufacture of a beer beverage, including the steps of:
- providing a starch material and converting the starch material into a sugar source;
- deriving a wort from the sugar source;
- subjecting the wort to steps of sparging and applying known filtration methods to the wort;
- boiling the wort and adding a flavouring agent in the form of plant material selected from the species *Aspalathus linearis* (rooibos), *Cyclopia* (honeybush), or a combination of these, or plant material of *Athrixia phylicoides* (Bush Tea) to the wort; and
- and thereafter subjecting the wort to a fermentation step, wherein said plant material is useful in imparting a unique flavour, aroma and mouthfeel to the beer beverage.

In this process hops can be replaced or the hops dosage can be reduced (or as an inverse function of hops dosage) so as to create the desired flavours, aromas and mouthfeel.
The invention further provides for extraction of polyphenols from the plant material being potentiated during the fermentation process, which polyphenols assist in the amplification of the flavour of the alcoholic beverage. Alternatively, the extraction of polyphenols derived from plant material may be potentiated during the step of boiling of the wort. The extracted polyphenols act as a natural preservative, thereby maintaining or possibly extending product shelf life.
The step of fermentation, alternatively the step of wort boiling, may increase the efficacy of polyphenol extraction and may occur over a temperature range of between 10 degrees Centigrade and 90 degrees Centigrade. Preferably, the step of fermentation may occur over a temperature range of between 10 degrees Centigrade and 20 degrees Centigrade.

The invention further provides that the plant material selected from the species *Aspalathus linearis* (rooibos), *Cyclopia* (honeybush), or a combination of these, or plant material of *Athrixia phylicoides* (Bush Tea) may include parts of the plant, including the roots, stems, leaves, branches, seeds, flowers, fruit and bark, or a combination of these. The plant material may further be in its natural form or may be in a processed form.
The sugar source may be starches and the starches may be selected from any one or more of malted cereal grains, such as barley or wheat, or a combination of the foregoing.
The invention may further provide that the polyphenols extracted from the plant material of the species *Aspalathus linearis* (rooibos), *Cyclopia* (honeybush), or a combination of these, or plant material of *Athrixia phylicoides* (Bush Tea) may include compounds from either of the groups, flavonoids or non-flavonoids. The flavonoids may include quercetin, luteolin, orientin, iso-orientin, vitexin, iso-vitexin and aspalathin, the latter being unique to *Aspalathus linearis.* In particular, the extracted polyphenols may include flavonoids, and may further include higher alcohols such as amylalcohols. The formed amylalcohols may contribute to an increased frutiness in the beer product, as hops dosage is replaced, or reduced (or as an inverse function of hops dosage. Described is an alcoholic beverage manufactured substantially according to the processes described hereinabove. The alcoholic beverage may be a beer product or a derivative thereof, more specifically a beverage containing at least a percentage of said beer product.

### BRIEF DESCRIPTION OF THE DRAWINGS

The invention will now be described by way of example only with reference to the accompanying drawings wherein:
- Figure 1: is a flow diagram of an improved process for the manufacture of an alcoholic wine beverage according to a second embodiment of the invention;
- Figure 2: is a flow diagram of an improved process for the manufacture of an alcoholic beer beverage according to a third embodiment of the invention.
- Figure 3: is a graphical depiction of extract reduction during a fermentation process in mature beer, measured as a function of time, across all samples mentioned in Example 2 below, inclusive of three control samples;
- Figure 4: is a graphical depiction of identified flavonoids extracted from mature beer during a lagering process, identified and quantified against a function of concentration; and

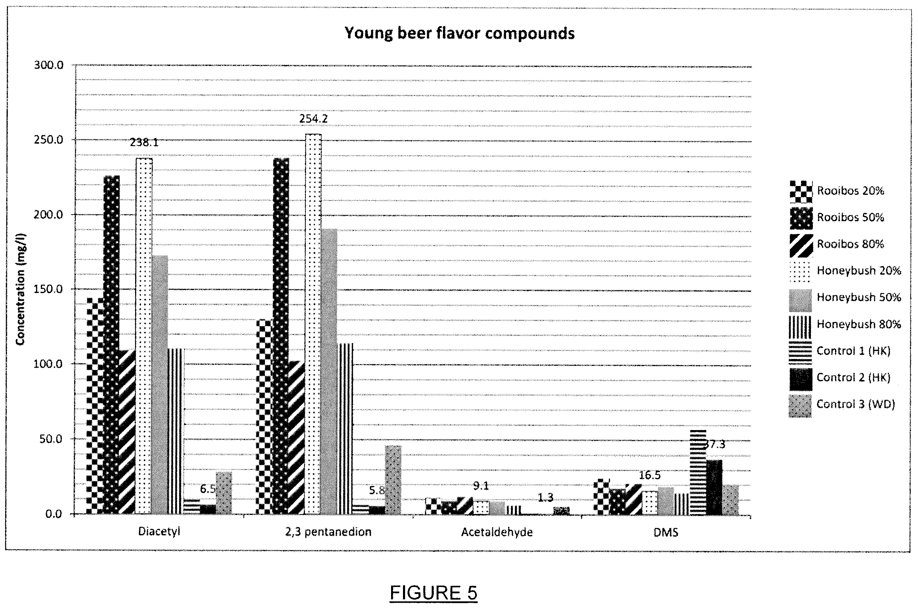

- Figure 5: is a graphical depiction of flavanoids extracted from young beer, identified and quantified as a function of concentration across samples mentioned in Example 2 below, inclusive of three control samples.

### DETAILED DESCRIPTION OF THE INVENTION

The quality of wines, specifically red wine, is judged from four criteria, namely flavour, aroma, colour and mouthfeel. In the highly competitive market of red wine products, the balancing of these three criteria to have universal appeal to the consumer market makes a successful product. Subject to local legislation, the wine-making process is usually highly regulated and the ability to introduce different tastes in wine is largely restricted.

The main anti-oxidant normally used in wine production is sulphur dioxide (SO₂). SO₂ in wine consists of free SO₂ and bound SO₂. The legal limit of total SO₂ in South African table wines is 150 mg/L. Bound SO₂ is normally bound to acetaldehyde or anthocyanins in red wine, but have little anti-oxidative or anti-microbial activity. However, the free SO₂ fraction in wine has the most anti-microbial and anti-oxidative activity in wine and is normally adjusted to about 35-40 mg/L at bottling. SO₂ has an anti-oxidative action by binding acetaldehyde generated from the oxidation of phenolic compounds in wine. SO₂ can also bleach brown quinones and eliminate H₂O₂ formed from the oxidation of phenolics in wine and is thus a very effective anti-oxidant in wine. However, the applicant has found it advantageous to lower SO₂ levels in wine due to health reasons. About 10% of the general population is thought to be allergic to SO₂ in wine and lowering the levels of this preservative could thus have a large benefit to the wine industry. An effective replacement for SO₂ in the wine industry has not been successfully implemented.

Wine producers will often mature red wine and certain white wines such as Chardonnay or Chenin blanc in wooden barrels. These barrels are normally produced from oak trees (Quercus species) which imparts favourable flavour compounds such as lactones (coconut and woody flavours), vanillin (vanilla flavours) and eugenol (spicy flavours) to the wine. Oak barrels are becoming increasingly expensive and wine producers are adopting the use of alternative oak products such as oak chips and wooden staves in recent years. The alternative oak market is thus growing and wine producers are looking for new products which could add value to their wine in terms of its flavour spectrum.

The Fabaceae family species *Aspalathus linearis* (Rooibos) and *Cyclopia* (Honeybush) species, or a combination of these or the plant material of the family Asteraceae, more specifically of Athrixia phylicoides (Bush Tea), is proposed as alternative to oak products mentioned above.

Both Rooibos and Honey Bush plant species are both indigenous to South Africa and have been proven to contain large amounts of phenolic compounds, such as quercetin, luteolin, orientin, iso-orientin, vitexin, iso-vitexin and aspalathin, which may act as anti-oxidants. The use of plant material (roots, wood, leaves and tea extracts) of the mentioned species as anti-oxidants or as a possible wood replacement has not, until now, been considered in the context of alcoholic wine beverage production. The plants of the mentioned species are known to impart health benefits, particularly in the form of tea. However, the use of these plants in red wine production as a sulphur substitute and its concomittent influence in the wine's flavour profile has, until now been unknown.

The applicant has thus found an improved process to augment the flavour, colour, mouthfeel and aroma of a red wine product (and other alcoholic beverages), and has surprisingly found that the addition of the flavour-improving additive has a natural preservative effect. The process also yields a product that may be useful as a tonic for health improvement, or as a useful and alternative prophylaxis. The invention will now be described with reference to the three examples that follow.

### Example 1 (Wine Beverage):

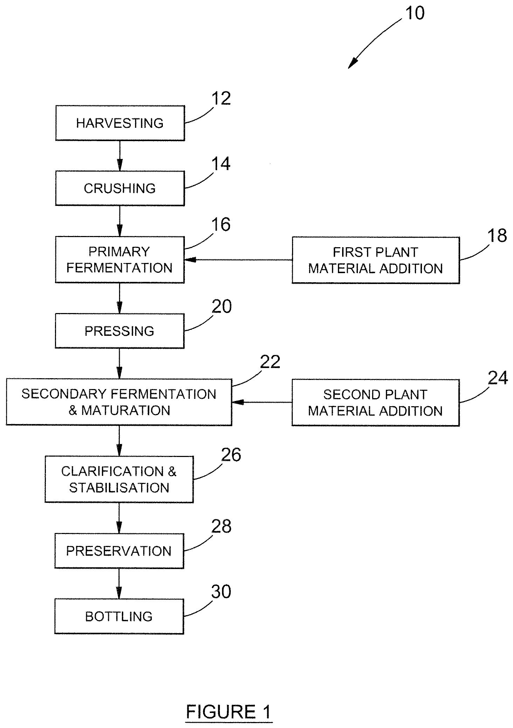

Referring to the Figure 1, an improved process for the manufacture of a low-sulphur alcoholic beverage according to a first aspect of the invention is generally designated by reference numeral 10.

The process of manufacture of the present invention includes the steps of harvesting 12 Shiraz grapes, crushing 14 the grapes to form a must and then subjecting the must to primary fermentation 16 in a suitable fermentation vessel.

During primary fermentation 16, the added specialized cultured yeast converts the natural sugars to alcohol. During this step, the primary fermentation 16 of the must causes a rise in temperature, from room temperature to approximately 20 to 30 degrees Centigrade. This temperature rise is responsible for the extraction of various polyphenols from the skin, stems and seeds of the grapes present in the must. Preferably, the step of (conventional) primary fermentation may occur over a temperature range of between 20 degrees Centigrade and 30 degrees Centigrade. However, through the use of existing technology the preference range could be much greater with a maximum of up to 90 degrees Centigrade.

Simultaneously with the step of primary fermentation 16, components of the plant *Aspalathus linearis* (Rooibos) are added to the must, with the cultured yeast. The temperature in the fermentation vessel is most preferably maintained at a temperature of 20 to 30 degrees Centigrade, until alcoholic fermentation is completed. This facilitates the extraction of additional polyphenols from the Rooibos, including some additional tannins, anti-oxidants and flavonoids. However, through the use of technology the preference range could be much greater with a maximum of up to 90 degrees Centigrade. This step of plant material addition is partly responsible for imparting a unique *Aspalathus linearis* flavour, mouthfeel, aroma and colour to the wine. Additionally, the presence of anti-oxidants in the must allows for a longer shelf life of the must, and (ultimately) the wine product produced by the process of manufacture. This allows a winemaker to greatly reduce the amount of artificial preservatives (specifically sulphur) that is typically added to the wine product, or to simply omit sulphur from the process altogether.

After primary fermentation 16 and first *Aspalathus linearis* plant component-additions 18, the must undergoes pressing 20 to liberate pressed juice from the crushed must and *Aspalathus linearis* plant additions, in addition to the free run juice already formed.

The wine is hereafter transferred to oak barrels or stainless steel tanks for secondary fermentation and maturation 22. During this step, strains of bacteria are added to induce Malolactic fermentation where malic acid gets converted to lactic acid.

Simultaneously with the step of secondary fermentation and maturation 22, a second *Aspalathus linearis* plant addition 24 is added to the wine. This second addition step 24 is optional and is for (1) the extraction of additional polyphenols so as to increase or enhance the distinct and unique *Aspalathus linearis* flavour of the wine, as well as improve upon the flavour, aroma and mouthfeel of the wine and (2) allow for the further reduction of the amount of artificial preservatives (specifically sulphur) that is typically added to the wine product, or to simply omit sulphur (whether in the form of sulphur dioxide or other suitable sources) from the process altogether

*Aspalathus linearis* has been the subject of extensive research and has proven to be rich in flavonoids, including various antioxidants. The presence of these useful compounds, in addition to the fact that *Aspalathus linearis* contains 0 % caffeine, may lead to various known health benefits. It has been documented that *Aspalathus linearis* may be useful as an immune-modulatory agent, a chemo-therapeutic agent, and an anti-oxidant agent.

As part of the secondary fermentation and maturation step 22, the wine is left in the barrels or tanks to mature for a period of between 6-30 months, during which time it undergoes routine laboratory sampling tests to monitor the progress of the maturation process, as well as intermittent tasting by the winemaker in order to assess the organoleptic progression of the wine.

Once maturation 22 is completed, the wine is treated with selected substances known in the art as part of a clarification and stabilisation if necessary step 26. Under the hitherto prior art process of manufacture, sulphur dioxide would if needed, at this stage be added to the wine, as part of the preservation step 28. However, due to the polyphenols (including anti-oxidants and flavonoids) extracted from the plant components of the *Aspalathus linearis* plant during primary and secondary fermentation, the amount of sulphur dioxide needed in the preservation step 28 is reduced. Typically, sulphur dioxide concentrations in the amount of up to 200 mg/l would be added under the conventional wine making process to ensure effective preservation, with an average concentration of 100 mg/l total sulphur dioxide in a typical red wine product. However, with the improved method of the current invention, a reduced sulphur dioxide concentration amounting to only approximately 50 mg/l total sulphur or less, being half of the conventional sulphur dioxide requirement, needs to be added during the preservation step 28. The said sulphur dioxide concentration may be reduced even further or omitted completely, depending on the wine used.

As a final step, the new wine product is bottled and ready for distribution.

### Experimental Wine Production

### Materials and methods

For this experiment a 2012 Shiraz wine was produced from Shiraz grapes sourced from the applicant in the Western Cape, South Africa. A sample of 250 kg of the Shiraz grapes were harvested and uniformly mixed to ensure homogeneity between different treatments defined below. The wines were produced on pilot scale using methods normally employed at the DVO to produce experimental wines. Grapes were de-stemmed, inoculated with *Saccharomyces cerevisiae* and fermented at 25°C. An addition of 0.5 g/L DAP was made on the second day of fermentation. Grapeskins were mixed with grape juice twice a day for colour and tannin extraction. Immediatley prior to yeast inoculation, the following treatments (20 kg each) were employed:
- SO₂ addition- 30 mg/L before fermentation and 50 mg/L after MLF;
- No addition of SO₂ or plant material;
- Rooibos (fermented)- added at 5 g/L, no addition of SO₂;
- Rooibos (fermented)- added at 10 g/L, no addition of SO₂;
- Honey Bush- added at 5 g/L, no addition of SO₂; and
- Honey Bush- added at 10 g/L, no addition of SO₂.

After alcoholic fermentation, the grape skins were pressed. The wines went through malolactic fermentation and were bottled in 750 mL glass bottles. Enhanced oxidation of these wines were then investigated. H₂O₂ is thought to act as an accelerated oxidation/ageing technique in wine, by addition. Different levels of H₂O₂ were added to wine samples on the postulate that SO₂ removal by H₂O₂ would provide a valid indication of oxidation status. Validation testing of this postulate indicated that 500 mg/L SO₂ is equivalent H₂O₂ dosage. This would roughly boil down to about a 125 mg/L O₂ addition, which is an over-estimation of what is observed in normal winemaking procedures. This dosage was employed to test the Rooibos and Honey Bush effect under extreme oxidation conditions.

The following wine treatments were exposed to H₂O₂ one week before the tasting started:
1) no SO₂ addition before fermentation and 80 mg/L SO₂ added just before treatment with H₂O₂;
2) No addition of SO₂ or plant material;
3) 30 mg/L SO₂ addition before fermentation and 50 mg/L at bottling;
4) Rooibos fermented- added at 5 g/L to must, no addition of SO₂;
5) Rooibos fermented- added at 10 g/L to must, no addition of SO₂;
6) Rooibos fermented- added at 10 g/L to must and additional 10 g/L added just before H₂O₂ exposure, no addition of SO₂;
7) Honey Bush- added at 5 g/L to must, no addition of SO₂;
8) Honey Bush- added at 10 g/L to must, no addition of SO₂; and
9) Honey Bush- added at 10 g/L to must and additional 10 g/L added just before H₂O₂ exposure, no addition of SO₂.

H₂O₂ was added to all of the above treatments and the wines were left at 20°C for 1 week before analyses and tastings were conducted.

### Sensory analyses

A trained tasting panel consisting of 9 persons were used. The method employed to conduct these tastings was Descriptive Analyses. After initial discussion on the samples the panel generated the following flavour descriptors for the wines: berry, dried fruit, prune, rose, black pepper, vanilla, rooibos, honey bush, green apple, sherry and sulphur compounds.

For most of the attributes two reference standards were presented to the panel for training purposes, one fresh reference and one soaked in a neutral red wine. These standards included fresh green apple, sherry wine, *Beta-*mercapto-ethanol (sulphur compound), fresh rose water, fresh red berries, dried fruit, dried prunes, black pepper seeds, vanilla pods, honey bush tea and rooibos tea. Natural product reference standards better represents the complexity of attributes as opposed to using a single chemical compound.

The taste testing was conducted in a well-ventilated sensory laboratory at a temperature of 20°C ± 2°C with individual booths. Dark ISO glasses were used with a sample volume of ± 30 ml. Lids were placed on each glass just after pouring to prevent aroma loss or contamination of the sensory laboratory. Pouring was done at the maximum 30 minutes before testing. A 120 mm unstructured line scale was used to assess the intensity of each attribute from "none" to "intense". Glasses were marked with three digit codes and samples randomized within each repetition. Purified water and unsalted crackers were used as palate cleansers. Samples were only evaluated based on aroma, not taste.

A total of 9 wine samples were thus evaluated using descriptive analysis by the trained panel in triplicate. Testing was done in three two hour sessions. 9 samples were tested per session in triplicate with a 15 minute break between repetitions to minimize sensory fatigue. A complete block design was used with randomization within repetitions; therefore all the samples were tested by all the judges. The judges rated the eleven attributes previously described per sample.

### Statistical analysis of tasting data

Panel performance was assessed using PanelCheck® software (Version 1.4.0, Nofima, As, Norway). Principle component analysis (PCA) was conducted using the mean data after weighing and centring was applied (Unscrambler X, version 10.1, CAMO Inc., Oslo, Norway). Analysis of variance (ANOVA) was used to investigate significant differences and interaction between the two chemical compounds and response surface graphs were obtained with (Statistica, version 10, Statsoft Inc., Tulsa, USA).

### Chemical analyses

The wine sample was observed for colour density, total red pigments and total phenolics spectrophotometrically, as well as acetaldehyde concentration immediately after the sensory analyses. The acetaldehyde concentrations were determined with the Kronelab robot using an enzymatic method.

### Results and Discussion

The acetaldehyde concentrations of the different treatments can be seen in Table 1.

**Table 1: Acetaldehyde values for the respective treatments**

| **Code** | **Treatment** | **(mg/L)** |
|---|---|---|
| 1 | +SO2 | 39.9 |
| 2 | -SO2 | 56 |
| 3 | + SO2 | 19.4 |
| 4 | RB 5 g/L | 55.6 |
| 5 | RB 10 g/L | 56.6 |
| 6 | RB 20 g/L | 34.4 |
| 7 | HB 5 g/L | 44.7 |
| 8 | HB 10 g/L | 42.5 |
| 9 | HB 20 g/L | 29.7 |

Acetaldehyde levels were the lowest where SO₂ was used (treatments 1 and 3). This was probably due to the ability of SO₂ to bind acetaldehyde or prevent its formation. The highest levels of acetaldehyde were found in treatment 2, with no additions. The addition of Rooibos or Honey Bush plant material led to lower levels of acetaldehyde than in treatment 3, with the 20 g/L Honey Bush addition leading to the lowest acetaldehyde levels. Acetaldehyde is an important indicator of oxidation and lower levels of this compound in wine is thus desirable.

Table 2 shows brown, red and purple colour fractions of the wine as well as colour density. Normally a red wine's colour density is between 10-20 AU, with a higher value indicating a more intense colour. The largest differences in treatments were observed at 520 nm, with smaller differences observed at 420 and 620 nm. Treatment 1 (SO₂ addition) showed the lowest colour density, due to the bleaching effect of SO₂ on the red flavilium ion (red coloured anthocyanin). Interesting enough where SO₂ was added at an earlier stage (treatment 3) it led to the highest colour density. This was also reflected in the total red pigments which can be seen in Table 2.

**Table 2: Brown (420nm), red (520nm) and purple (620nm) absorbency units as well as the colour density (sum of 420+520+620nm) of the red wines after the treatments.**

| **code** | **Treatment** | **420 nm (AU)** | **520 nm (AU)** | **620 nm (AU)** | **Colour density (AU)** |
|---|---|---|---|---|---|
| 1 | +SO2 | 3.20 | 5.12 | 1.12 | 9.44 |
| 2 | -SO2 | 4.37 | 6.79 | 1.66 | 12.82 |
| 3 | + SO2 | 4.51 | 8.28 | 1.61 | 14.41 |
| 4 | RB 5 g/L | 4.35 | 6.69 | 1.60 | 12.64 |
| 5 | RB 10 g/L | 4.54 | 6.74 | 1.65 | 12.93 |
| 6 | RB 20 g/L | 4.24 | 5.37 | 1.37 | 10.97 |
| 7 | HB 5 g/L | 4.73 | 7.31 | 1.77 | 13.81 |
| 8 | HB 10 g/L | 4.62 | 6.86 | 1.71 | 13.19 |
| 9 | HB 20 g/L | 4.01 | 5.29 | 1.26 | 10.55 |

The total quantum of red pigments (Table 3) is measured at a very low pH value. In other words all the anthocyanins and colour pigments in the wine are forced into the red form. It thus indicates a pool of potential colour of the wine which will develop during ageing, with values normally ranging in red wine at 10-30 AU. During ageing of red wine this value can decrease due to polymerisation and precipitation of the coloured pigments. Higher plant material additions (treatments 6 and 9) led to lower total red pigment coloured values, which could be due to over polymerisation and precipitation. However, the addition of H₂O₂ led to a higher % of colour in the red form, except where SO₂ was added (treatments 1 and 3), which is probably due to the effect of SO₂ preventing acetaldehyde formation and the subsequent phenolic polymerisation and increase in colour due to colour pigment formation. It thus seems that the addition of the Rooibos and especially the Honey Bush led to increased polymerisation of phenolic compounds. This was also reflected in the total phenolics (Table 3), which led to lower values due to probable over polymerisation and precipitation.

**Table 3: Total phenol, total red pigment and % of colour in the red form of the red wines after the treatments.**

| **code** | **Treatment** | **Total Phenols (AU)** | **Total red pigments (AU)** | **% of colour in the red form** |
|---|---|---|---|---|
| 1 | +SO2 | 54.93 | 27.59 | 34 |
| 2 | -SO2 | 47.51 | 17.77 | 72 |
| 3 | + SO2 | 53.41 | 25.53 | 56 |
| 4 | RB 5 g/L | 49.86 | 17.67 | 72 |
| 5 | RB 10 g/L | 49.00 | 18.19 | 71 |
| 6 | RB 20 g/L | 50.58 | 15.21 | 72 |
| 7 | HB 5 g/L | 48.17 | 19.3 | 72 |
| 8 | HB 10 g/L | 50.11 | 19.36 | 68 |
| 9 | HB 20 g/L | 47.79 | 15.32 | 69 |

Sensory descriptive analyses is a sensory tool often used in food science to quantitatively distinguish between different treatments. Although a large number of chemical compounds can already be measured in wine, sensory analysis is probably still the most powerful tool to distinguish oxidation levels in wines. Treatments 4 and 5 are mostly associated with prune and vanilla aroma, while treatment 6 is associated mostly with dried fruit and rooibos descriptors. Treatment 8 and 9 are associated more with honey bush, rose and black pepper aromas. Treatments 1 and 3 are associated mostly with sulphur compounds, while treatment 2 is associated mostly with sherry and green apple flavours.

The berry attribute was generally low in most treatments, except in treatment 3 (+S02) and treatment 7 (HB 5 g/L). The Rooibos aroma was highest in treatment 6 (RB 20g/L), with treatments 4 and 5 (5 g/L and 10 g/L RB) still showing Rooibos aromas. The Honey Bush aroma was the highest in treatment 9 (HB 20 g/L), with lower levels observed in treatments 7 and 8 (5 g/L and 10 g/L HB). Some Rooibos treatments led to higher vanilla and dried fruit aromas, while the Honey Bush treatments led to higher black pepper and especially rose aromas being perceived by the panel. Treatment 2 (-SO2) also had significantly higher green apple and sherry aromas than the other treatments. Low levels of green apple and sherry were also perceived in treatments 7 (HB 5 g/L). The other treatments showed very low levels of these aromas in the wines. The SO₂ treatments (1 and 3) showed a negative reductive sulphur linked aroma, which was not perceived in the other treatments.

It thus seems that Rooibos enhances the vanilla and Rooibos aromas while Honey Bush that of honey bush, roses and black pepper.

Interestingly enough the addition of the plant material also led to lower levels of the oxidative derived green apple and sherry-like aromas. Although acetaldehyde levels were in some cases lower where higher levels of plant material was used (treatments 6 and 9), concentrations of this compound did not differ drastically in the lower plant material additions treatments (3, 5, 7 and 8) from treatment 3. However, these treatments were still perceived by the panel to be lower in green apple and sherry aromas normally associated with this compound. This could indicate a possible anti-oxidant capacity of these types of plant materials or a masking effect of the aromas associated with these plant materials over the oxidation derived aromas. None of the plant material treated wines were also rated as being faulty in any way by the panellists indicating it could be used in wine.

It should be kept in mind that the wines in this experiment were exposed to high levels of oxidation and this work should be repeated in wines with more industry related levels of oxidation. In the final report we will report on the sensory and chemical analyses of the other experiments, which included ageing of Audacia wines in barrels as well as additional wines exposed to oxidation. Rooibos and Honey Bush plant material might thus have some anti-oxidant capacity in wine, as well as serve as an alternative wood (oak) replacement, but follow on research is required.

### Example 2 (beer):

In this specification, the following terms and abbreviations are used:

| **TERM / ABBREVIATION** | **DEFINITION** |
|---|---|
| BU | Bitter Units |
| CTA | Chemical Technical Analysis |
| EBC | European Brewery Convention |
| GC | Gas Chromatography |
| OG | Original Gravity |

It is well-known that plants of the Fabaceae family, indigenous to southern Africa, enjoy recognition as versatile and useful health products. These plants, in particular *Aspalanthus linearis* (Rooibos or RB), *Cyclopia spp.* (Honeybush) and *Athrixia phylicoides* (African Bush Tea) are rich in polyphenolic compounds and other extracts, each of which hold certain health benefits. Plant material of the mentioned plants (herein referred to as Fabaceae plant material 60) are used in the manufacture of beer in order to, *inter alia,* augment the taste of the final beer product 64. Tea, prepared separately, is known to be added to existing beer products. However, until now, not much was known on how polyphenolics, tannins and other compounds react with the beer product and how this may influence taste profiles of the beer product.

In addition to taste augmentation, certain polyphenolics that are natural preservatives may also act to enhance the shelf life of beer products, when used as a functional additive to the beer product. Hops is also a source of polyphenolics and simultaneously acts as a bittering agent when added to beer during beer production. Fabaceae plant material as used in the manufacture of beer according to the method set out further below, is used as a hops substitute, in varying degrees, in order to impart a palatable and novel flavour to beer, and thereby achieve a novel beer product. The degrees of substitution are as set out in Table 4 below:

**Table 4: Addition of Fabaceae Material During Hot Wort Preparation, Showing Degrees of Hops Substitution**

| **Hot Wort Preparation** | **Hops 80%** | | **Hops 50%** | | **Hops 20%** | | **Hops 0%** | |
|---|---|---|---|---|---|---|---|---|
| | 4 g/l | 8 g/l | 4 g/l | 8 g/l | 4 g/l | 8 g/l | 4 g/l | 8 g/l |
| **Rooibos Raw Material (fermented)** | X | X | X | X | X | X | X | X |
| **Honeybush** | X | X | X | X | X | X | X | X |

The process of manufacture of beer set out below has been prepared as a pilot scale experiment, the results of which appear further hereunder, and which experiment included hot wort preparation, and fermentation and lagering. Fabaceae substitution is provided for under varying degrees of hops substitution, shown in Table 4.

### Methodology

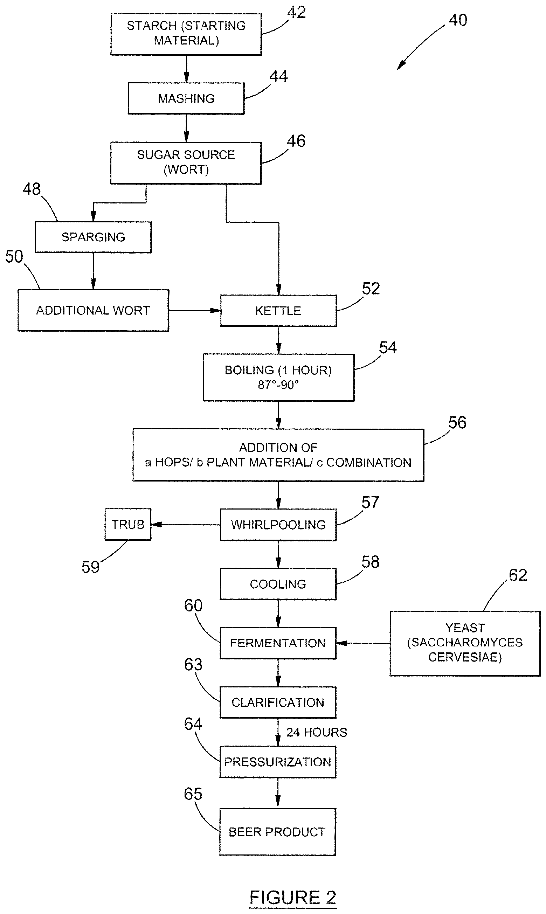

Referring now to Figure 2, an improved process for the manufacture of an alcoholic beverage according to the invention is generally designated by reference numeral 40.

A starch 42 starting material is provided, typically being in the form of malted cereal grains such as barley or wheat. The starch material 42 is converted into a sugar source 46, after undergoing a process known as mashing. The sugar source 46 typically takes the form of a sugary liquid called a wort (and these will be referred to interchangeably in the remainder of this specification). The wort 46 is prepared by mixing the starch material 42 (in a crushed form known as grists) with hot water by in a mashing step 44 for about 2 (two) hours.

The wort 46, is thereafter transferred to the wort kettle. Additional fermentable wort 50 is obtained through a further step known as "sparging" 48, wherein the remaining extract (note: starch converted to fermentable sugar) is washed out. Filter frames are used to separate the wort 46 and the mashed starch material during sparging 48.

Approximately 12 (twelve) litres of the filtered wort 46 collected as a result of the mashing and sparging steps are then placed in a pilot brewing plant kettle 52 (or so-called wort kettle). The filtered wort 46 and additional wort 60 is then brought to a boil at a temperature of between 90°C and 100°C. At this point, hops according to the dosage regime set out in Table 1 is added to the kettle in order to allow isomerisation of hop acids, together with the desired amount of Fabaceae plant material 60 by way of addition to the kettle 52. In this example, the Fabaceae plant material is available as different fractions of Rooibos plant material in the form of roll sieve stick, granules, light grade and DFC grade. Any of the mentioned forms is suitable in conducting the process according to example 2.

The wort 46 is thereafter boiled for 10 minutes before being subjected to a whirlpooling step 57 in order to separate trub 59. It will be readily appreciated that trub includes remnants of Fabaceae plant material 56 from which Fabaceae extract (not shown) has been obtained. The boiled and treated wort (not shown) is now subject to wort cooling 58 as is known in the art.

When the cooled wort (not shown) reaches a temperature of between 10 - 15°C, it is then transferred into a stainless steel container to begin a fermentation step 60. Yeast 62 (such (*Saccharomyces cerevisiae*) 0.005ml/ml wort (v/v) is added to 300ml of cold wort. This is then added to wort in the fermenter in fermentation step 60, as is known in the art. The fermenter is subjected to agitation in order to allow for complete yeast suspension. A total of 24 hours is allowed to elapse whereafter the fermenter is pressurized 64 to 1 bar counter pressure. Continued pressure and fermentation rate is closely monitored for approximately 8 to 9 days at a constant temperature of 10 - 13°C. This simultaneously allows the product to clarify.

As soon as the process of fermentation and clarification is complete, the beer product is transferred to a second fermenter under a carbon dioxide atmosphere and beer is allowed to mature at a temperature of 0°C. Once matured, the beer product is ready for filtration and thereafter packaging, labelling and sale.

### Measurements and Analysis

An analysis of the following variables was undertaken:
- standard CTA (end of primary fermentation);
- BU Analysis (hops yield);
- Monitoring of fermentation rate;
- GC Analysis (Lagering);
- Sensory Analysis.

### Observations and Results

The results of the pilot scale experiment are set out in the series of Tables below. These results include a tabularization of process parameters used, as described in more detail above, and summarized below, showing each degree of hops substitution with Fabaceae plant material. Additionally, analytics relating to the measurement of BU and OG is also detailed, based on testing of Honey Bush and Rooibos Fabaceae material. Tables 4 to 18 relate to fermented beer.

**Table 5: Process Parameters: 80% Hops Substitution with 8g/l Fabaceae Plant Material**

| Parameter | Honeybush | Rooibos |
|---|---|---|
| Volume of wort (I) | 12 | 12 |
| Mass of Fabaceae material (g) added | 8 g/l | 8g/l |
| Volume of hops (g) | 4.44 | 4.41 |
| Volume of yeast (ml) | 60 | 60 |
| Wort cooling temperature (°C) | 12 - 13 | 12 - 13 |
| Fermentation temp (°C) | 10 - 15 | 10 - 15 |
| Fermentation pressure (bar) | 1 | 1 |

**Table 6: Analytics for Honeybush material (cold wort) based on Table 1 Parameters**

| **Time (min) elapsed** | **Temp (°C)** | **Bitter Units (EBC)** | **OG(%)** | **pH** |
|---|---|---|---|---|
| 0 | 91 | 3.23 | 11.28 | 5.57 |
| 20 | 91 | 16.87 | n/a | n/a |
| 40 | 91 | 18.98 | n/a | n/a |
| 60 | 90 | 21.91 | 14.54 | 5.35 |

**Table 7: Analytics for Rooibos Plant Material based on Table 1 Parameters**

| **Time (min) elapsed** | **Temp (°C)** | **Bitter Units (EBC)** | **OG(%)** | **pH** |
|---|---|---|---|---|
| 0 | 89 | 3.23 | 11.28 | 5.57 |
| 20 | 90 | 16.35 | n/a | n/a |
| 40 | 91 | 18.97 | n/a | n/a |
| 60 | 89 | 24.54 | 14.36 | 5.39 |

**Table 8: 50% Hops Substitution with 8g/l Fabaceae Plant Material**

| **Parameter** | **Honeybush** | **Rooibos** |
|---|---|---|
| Volume of wort (I) | 12 | 12 |
| Mass of Fabaceae material (g) added | 96.13 | 96.10 |
| Mass of hops (g) | 2.76 | 2.76 |
| Volume of yeast (ml) | 60 | 60 |
| Wort cooling temperature (°C) | 12 - 13 | 12 - 13 |
| Fermentatiion temp (°C) | 10 - 15 | 10 - 15 |
| Fermentation pressure (bar) | 1 | 1 |

**Table 9: Analytics for Honeybush Plant Material based on Table 5 Parameters**

| **Time (min) elapsed** | **Temp (°C)** | **Bitter Units (EBC)** | **OG(%)** | **pH** |
|---|---|---|---|---|
| 0 | 90 | Not taken | 10.97 | 5.71 |
| 20 | 91 | 14.11 | n/a | n/a |
| 40 | 90 | 14.96 | n/a | n/a |
| 60 | 90 | 18.63 | 13.95 | 5.42 |

**Table 10: Analytics for Rooibos Plant Material based on Table 5 Parameters**

| **Time (min) elapsed** | **Temp (°C)** | **Bitter Units (EBC)** | **OG(%)** | **pH** |
|---|---|---|---|---|
| 0 | 91 | Not taken | 10.97 | 5.71 |
| 20 | 90 | 15.07 | n/a | n/a |
| 40 | 90 | 15.51 | n/a | n/a |
| 60 | 90 | 20.33 | 14.22 | 5.48 |

**Table 11: Process Parameters: 50% Hops Substitution with 4g/l Fabaceae Plant Material**

| **Parameter** | **Honeybush** | **Rooibos** |
|---|---|---|
| Volume of wort (I) | 20 | 20 |
| Mass of Fabaceae material (g) added | 80.18 | 80.23 |
| Mass of hops (g) | 4.59 | 4.59 |
| Volume of yeast (ml) | 100 | 100 |
| Wort cooling temperature (°C) | 12 - 13 | 12 - 13 |
| Fermentatiion temp (°C) | 10 - 15 | 10 - 15 |
| Fermentation pressure (bar) | 1 | 1 |

**Table 12: Analytics for Honeybush Based on Table 8 Parameters**

| **Time (min) elapsed** | **Temp (°C)** | **Bitter Units (EBC)** | **OG(%)** | **pH** |
|---|---|---|---|---|
| 0 | 90 | 2.89 | 11.53 | 5.63 |
| 20 | 91 | 11.41 | n/a | n/a |
| 40 | 90 | 12.21 | n/a | n/a |
| 60 | 90 | 12.09 | 13.16 | 5.53 |

**Table 13: Analytics for Rooibos Based on Table 8 Parameters**

| **Time (min) elapsed** | **Temp (°C)** | **Bitter Units (EBC)** | **OG(%)** | **pH** |
|---|---|---|---|---|
| 0 | 91 | 3.55 | 10.83 | 5.61 |
| 20 | 90 | 9.73 | n/a | n/a |
| 40 | 90 | 11.21 | n/a | n/a |
| 60 | 90 | 14.26 | 13.18 | 5.49 |

**Table 14: Process Parameters: 20% Hops Substitution with 8g/l Fabaceae Plant Material**

| **Parameter** | **Honeybush** | **Rooibos** |
|---|---|---|
| Volume of wort (l) | 12 | 12 |
| Mass of Fabaceae material (g) added | 96.09 | 96.14 |
| Mass of hops (g) | 1.14 | 1.11 |
| Volume of yeast (ml) | 60 | 60 |
| Wort cooling temperature (°C) | 12 - 13 | 12 - 13 |
| Fermentatiion temp (°C) | 10 - 15 | 10 - 15 |
| Fermentation pressure (bar) | 1 | 1 |

**Table 15: Analytics for Honeybush Based on Table 11 Parameters**

| **Time (min) elapsed** | **Temp (°C)** | **Bitter Units (EBC)** | **OG(%)** | **pH** |
|---|---|---|---|---|
| 0 | 90 | 3.60 | 11.19 | 5.56 |
| 20 | 90 | 7.89 | n/a | n/a |
| 40 | 91 | 7.95 | n/a | n/a |
| 60 | 90 | 10.68 | 14.42 | 5.34 |

### Analytics: Fabaceae variety Rooibos

**Table 16: Analytics for Rooibos Based on Table 11 Parameters**

| **Time (min) elapsed** | **Temp (°C)** | **Bitter Units (EBC)** | **OG(%)** | **pH** |
|---|---|---|---|---|
| 0 | 89 | 3.60 | 11.19 | 5.56 |
| 20 | 90 | 6.88 | n/a | n/a |
| 40 | 91 | 7.26 | n/a | n/a |
| 60 | 89 | 9.86 | 14.54 | 5.37 |

**Table 17: Process Parameters: 0% Hops Substitution with 8g/l Fabaceae Plant Material**

| **Parameter** | **Honeybush** |
|---|---|
| Volume of wort (l) | 20 |
| Mass of Fabaceae material (g) added | 160.00 |
| Mass of hops (g) | 0.00 |
| Volume of yeast (ml) | 100 |
| Wort cooling temperature (°C) | 12 - 13 |
| Fermentation temp (°C) | 10 - 15 |
| Fermentation pressure (bar) | 1 |
| OG before boil (%) | 11.85 |
| OG after boil (%) | 13.67 |

**Table 18: Process Parameters - Malt Beer 50% Hops Substitution with 4g/l Fabaceae Plant Material**

| **Parameter** | **Honeybush** |
|---|---|
| Volume of wort (l) | 17 |
| Mass of Fabaceae material (g) | 30 |
| Mass of hops (g) | 3.50 |

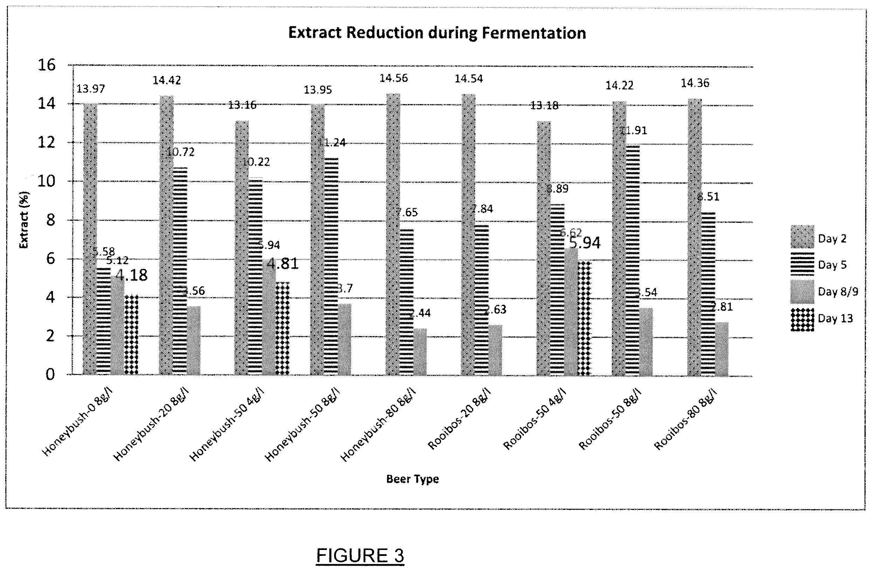

A graph showing fermentation concentrations of each of the mentioned Fabaceae material tests conducted is shown as Figure 3. The samples were measured on the basis of extract percentage against 13 days of observation.

Esters and higher alcohols were extracted and measured as a function of concentration. These values are set out in Table 19 below. These higher alcohol extracts were measured for each of the process parameters as listed in Tables 4 - 18, and identified the following compounds:
- Ethylacetate;
- n-Propanol;
- iso-Butanol;
- iso-Amylacetate; and
- amylalcohols.

Three samples of commercially available beer products were used as individual controls.

**Table 19: Values of Mature Beer Flavor Compounds shown Graphically in Figure 3**

| **Component** | **R-20** | **R-50** | **R 80** | **H-20** | **H-50** | **H-80** | **Control 1 (HK)** | **Control 2 (HK)** | **Control 3 (WD)** |
|---|---|---|---|---|---|---|---|---|---|
| Ethylacetate | 19.9 | 15.627 | 24.594 | 15.595 | 19.738 | 17.773 | 13.84 | 27.705 | 13.293 |
| n-Propanol | 21.034 | 16.774 | 16.469 | 16.824 | 15.297 | 14.038 | 14.304 | 12.133 | 9.084 |
| Iso-Butanol | 25.763 | 20.896 | 21.611 | 20.434 | 17.925 | 15.561 | 18.033 | 17.283 | 9.65 |
| Iso-Amylacetate | 1.969 | 1.78 | 2.705 | 1.755 | 2.382 | 2.411 | 1.644 | 4.418 | 1.489 |
| Amy alcohols | 93.708 | 83.18 | 87.692 | 82.19 | 73.275 | 68.906 | 64.189 | 73.227 | 50.454 |

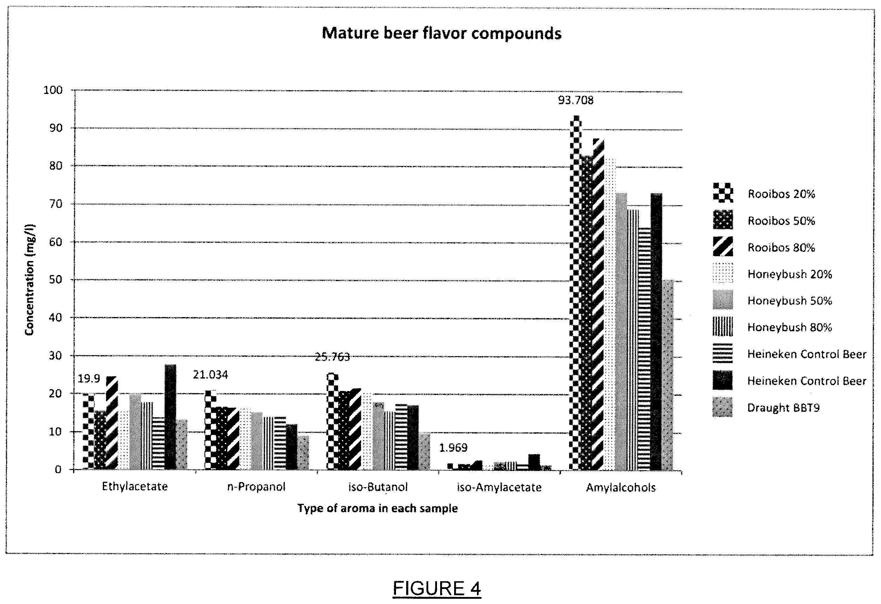

Gas chromatography was also used to determine flavanoid concentration (in mg/l) of young beer samples, the results of which is set out in Table 20. As above, three control samples were used based on commercially available beer products. These results are graphically represented in Figure 4.

**Table 20: Gas chromatography results of young beer, showing identified flavanoids**

| **Component** | **R-20** | **R-50** | **R-80** | **H-0** | **H-20** | **H-50** | **H-80** | **Control 1 (HK)** | **Control 2 (HK)** | **Control 3 (WD)** |
|---|---|---|---|---|---|---|---|---|---|---|
| Diacetyl | 144.1 | 226.3 | 109.2 | | 238.1 | 173.0 | 110.5 | 9.8 | 6.5 | 28.4 |
| 2,3-Pentanedion | 130.3 | 238.4 | 102.6 | | 254.2 | 191.2 | 114.2 | 6.5 | 5.8 | 46.0 |
| Acetaldehyde | 11.1 | 9.1 | 11.7 | | 9.1 | 8.7 | 6.1 | 1.6 | 1.3 | 5.4 |
| DMS | 24.2 | 17.4 | 20.7 | | 16.5 | 18.6 | 14.2 | 56.9 | 37.3 | 19.5 |
| Ethylacetate | 19.9 | 15.6 | 24.6 | | 15.6 | 19.7 | 17.8 | 13.8 | 27.7 | 13.3 |
| n-Propanol | 21.0 | 16.8 | 16.5 | | 16.8 | 15.3 | 14.0 | 14.3 | 12.1 | 9.1 |
| Iso-Butanol | 25.8 | 20.9 | 21.6 | | 20.4 | 17.9 | 15.6 | 18.0 | 17.3 | 9.7 |
| Iso-Amylacetate | 2.0 | 1.8 | 2.7 | | 1.8 | 2.4 | 2.4 | 1.6 | 4.4 | 1.5 |
| Amyalcohols | 93.7 | 83.2 | 87.7 | | 82.2 | 73.3 | 68.9 | 64.2 | 73.2 | 50.5 |

The final products obtained as a result of the pilot experiment were then subjected to peer-reviewed sensory analysis. Conclusions derived from that analysis are summarised in Table 21, based on findings of each sample produced. It is clear that amylalcohols contribute to a "fruitiness" flavour of the beer products tested. This flavour is pronounced as hops dosage is reduced.

**Table 21: Results of Sensory Analysis**

| **Dosage** | **80% hops + 8 g/l Fabaceae** | **50% hops + 8 g/l Fabaceae** | **50% hops + 4 g/l Fabaceae** | **20% hops + 8 g/l Fabaceae** | **0% hops + 8 g/l Fabaceae** |
|---|---|---|---|---|---|
| **Supporting explanation** | High Hops + High Fabaceae dosage | Medium Hops + High Fabaceae dosage | Medium Hops + medium Fabaceae dosage | Low Hops + High Fabaceae dosage | No hops + High Fabaceae dosage |
| Taster remarks | Tea taste over-pronounced | Strong bitterness | Pleasant fruitiness, Pleasant tea after taste | Pronounced fruitiness and tea taste | Pronounced tea taste |

### Cider

### Principles of Fermentation

Cider is made from apple juice which has undergone two different kinds of fermentation. The first fermentation is carried out by yeasts which have either been added deliberately or which are naturally present on the apple skins. This fermentation converts sugars to ethanol, esters and the higher alcohols *(fusel alcohols).* The second fermentation, the *malo-lactic fermentation* converts L(-)-malic acid to L(+)-lactic acid and carbon dioxide. This fermentation is carried out by lactic acid bacteria which are present in the apple juice and also in the area in which the fermentation is carried out. The malo-lactic fermentation can occur concurrently with the yeast fermentation but more often it is delayed until the fully fermented cider reaches 15°C, normally in the late spring or early summer of the year following that in which the cider was made.

### The Cider Making Process

Traditional cider making starts with the picking of the apples. These are left to mature for a week and then tipped into a "scratcher" which crushes the apples. In more modern plants the apples are reduced to a pulp in a grater type mill made of stainless steel. The apple pulp is known as "pomace" or "pommy".

Next the pulp is crushed to extract juice. This is done in a cider press. Several types of presses are used. The traditional type is a rack and cloth press (sometimes known as a "pack press"). In this type of press a sheet of sisal or hessian is placed across the bottom of a square frame above a trough. A layer of pomace, 4-5 inches deep, is poured onto the hessian. The hessian is folded over the pomace, completely enclosing it. Another sheet of hessian is placed on top of the first and the process repeated until the layers fill the frame. The cider press is then racked down onto the layers and the juice runs into the trough. The pomace is pressed until it is solid and no more juice runs out. The press is then racked up, the layers of pomace are broken up by hand, and the whole lot is re-pressed. In modern plants mechano-hydraulically operated plate presses are used.

Freshly pressed juice may be fermented straight away. In some commercial operations it is concentrated and stored for later conversion to cider, in which case it is extensively treated to pasturise it and to remove pectin. The fresh juice may be fermented in one of two different ways. Traditionally the juice is run into a wooden pipe (a barrel which can contain 120 gallons) or smaller wooden barrels, and the bung of the barrel is removed. No yeast is added, traditional cider making relies on wild yeasts (or wild yeast fermentation). The fermentation (wild yeast fermentation) starts in 1-2 days and continues for several weeks, during which time the barrel is topped up with more cider. When fermentation is over, the bung is replaced and the cider matured for 5-6 months. The process of manufacture in terms of the invention includes the further step of simultaneously adding plant material selected from the species *Aspalathus linearis* (rooibos), *Cyclopia* (honeybush), or a combination of these, or plant material of *Athrixia phylicoides* (Bush Tea) during the step of wild yeast fermentation, thereby potentiating extraction of polyphenols from the plant material, useful in imparting a unique flavour and aroma to the cider.

The step of fermentation may increase the efficacy of polyphenol extraction in a similar fashion to that described in respect of wine and beer above.

There may be further provided, according to the invention, that the process of manufacture of low sulphur cider may include a further and/or alternative step of optionally adding plant material selected from the species *Aspalathus linearis* (rooibos), *Cyclopia* (honeybush), or a combination of these, or plant material of *Athrixia phylicoides* (Bush Tea) during a secondary fermentation step (malolactic fermentation which occurs through the addition of cultured yeast). The secondary fermentation step may precede the step of applying known methods of clarification, stabilisation, fining, and filtration to the apple juice, in order to produce a cider product.

Alternatively the juice can be treated with the plant material selected from the species *Aspalathus linearis* (rooibos), *Cyclopia* (honeybush), or a combination of these, or plant material of *Athrixia phylicoides* (Bush Tea) to reduce oxidation and reduced levels of sulphur dioxide added to inhibit wild yeast fermentation, and then fermented with added cultured yeast. This method can be used in high output commercial operations. After the initial fermentation subsides, the cider is left for the yeast to settle, and it is either racked and/or centrifuged and placed into storage tanks. Storage may last 12-18 months, and the cider is blended with new and old ciders to moderate any excessive changes thus maintaining a consistent flavour profile year on year. These cider blends are nearly always cleared by centrifugation or kieselguhr filtration. This type of cider is sterilised by sterile filtration or flash pasteurization and is artificially carbonated in the bottle by counter-pressure bottle fillers. Plant material or reduced levels of sulphur dioxide can be added at this stage to maintain the stability of the cider. The resulting product may be considered analagous to keg beer.

### Characteristics of Apple Juice

Compared to wort, apple juice has a much lower pH, a much lower soluble nitrogen content, and a virtual absence of any sugars other than mono- and di-saccharides. The composition of the juice varies with the apple variety used. The average composition of cider apple juice in terms of its sugar content is 74% fructose, 15% sucrose, and 11% glucose. There are almost no other sugars present so that there is very little residual gravity left in fully-fermented ciders.

The major acid present is L(-)-malic acid but shikimic, quinic, chlorogenic and *p*-coumarylquinic acids are commonly present. The juice also contains soluble pectin (polymers of galacturonic acid esterified with methanol). Tannins are present, mainly epi-catechin, dimeric and trimeric pro-anthocyanidin and phenolic acids. These phenolics are the fraction which undergoes oxidation in damaged fruit.

The soluble nitrogen content is low and is largely made up of asparagine, aspartic and glutamic acids. Apple juice usually contains one eighth of the soluble nitrogen content of wort. The lower nitrogen content is further exaggerated by much lower pitching rates used in cider making when compared to beer making, usually 5-15 times lower. This means that the apple juice must support a higher degree of yeast growth and thus the fermentation is much protracted. Some commercial operations now add ammonium sulphate to the cider to give rapid and consistent fermentations. This may not be necessary when Fabaceae plant material is added to the cider.

### The Microbiology of Apple Juice

Ripe apples have less than 500 yeast-like organisms per g of sound fruit. The main organisms are *Aureobasidium pullulans, Rhodotorula spp., Torulopsis, Candida, Metschnikowia,* and *Kloeckera apiculata. Saccharomyces* species and other sporulating yeasts are rarely found. Acid-tolerant bacteria such as *Acetomonas spp.* are usually present. Lactic-acid bacteria are rare. The amounts of micro-organisms rise if the fruit is allowed to fall naturally or particularly if the skin is damaged. Yeast counts rise due to the indigenous flora of the factory in which the apples are processed. The traditional rack and cloth press is also a major source of contamination.

Apple juice cannot be sterilised by heating since the pectin esterase enzymes in the juice are destroyed by heat, thus the resulting cider will not clear. The addition of sulphur dioxide has been the most common way of controlling unwanted organisms. The amount of sulphur dioxide needed depends on the pH of the juice. Between pH 3.0 to 3.3, 75 ppm is needed, between pH 3.3 and 3.5 100 ppm is necessary and 150 ppm between 3.5 and 3.8. In the UK the maximum legal limit for sulphur dioxide is 200 ppm and this may well be lowered by subsequent legislation. The sulphur dioxide can be added in the form of Campden tablets. The juice is left overnight to allow the different forms of dissolved sulphur dioxide to equilibrate. Aerobic yeasts, and lactic and acetic acid bacteria are generally destroyed. The activity of other yeasts is usually inhibited. If there were substantial amounts of rotten fruits used to make the juice, compounds present in these fruits such as 2,5-D-*threo*-hexodiulose and 2,5-diketogluconic acid will strongly inhibit the action of the sulphur dioxide. As well as preventing infections, the sulphur dioxide also has an anti-oxidant function producing a cleaner flavour. This is not necessarily an advantage, the use of sulphur dioxide has led to sweeter ciders with a loss of the apple character in the flavour.

The malo-lactic fermentation is carried out by non-slime forming strains of *Leuconostoc mesenteroides, Lactobacillus collinoides* and very rarely *Pediococcus cerevisiae.* These bacteria are readily inhibited by the levels of sulphur dioxide used in cider making yet ciders readily undergo malo-lactic fermentation in the spring/summer after they were made. The explanation for this is not certain, possibly lab strains of these organisms are more sensitive to sulphur dioxide than are wild strains, possibly the sulphur dioxide merely inhibits the bacteria and they subsequently recover, or possibly there are other organisms at work.

### Changes in Apple Juice Composition During Fermentation and Maturation

The ciders mentioned are fermented with naturally occurring yeasts. It is assumed, but not known, that similar processes occur when fermentation with pure cultures is used.

At the end of the yeast fermentation, yeast release nitrogenous compounds into the cider. These include amino acids and peptides. Pantothenic acid and riboflavin are also released along with some phosphorus compounds. The release of nutrients is important since it is necessary for the malo-lactic fermentation to occur.

During the yeast fermentation there is an increase in acidity due to the formation of L(-)-malic acid by the yeast. Gluconic, lactic and succinic acids are also formed. Mono- di- and tri-galacturonides are present from the enzymic degredation of pectin, and keto acids are also formed. Higher or fusel alcohols are formed; unlike beer where they are unwanted compounds, in cider they form important components of the flavour profile. The levels formed depend on apple variety, juice treatment, yeast strain, fermentation conditions, and storage conditions. In general, low pH and low nitrogen levels tend to produce ciders with higher fusel alcohol levels. Use of sulphur dioxide, and centrifugation of the apple juice before fermentation both result in the lowering of fusel alcohol levels. The factor most affecting fusel alcohol levels is the strain of yeast. Aeration is also a factor, aeration reduces fusel production markedly.

The maturation phase of cider production includes the malo-lactic fermentation. In this stage, malic acid is converted to lactic acid and carbon dioxide. The exact type of acid produced depends on pH. At pH 3.6 more lactic than succinic acid is produced, whilst at pH 4.8 only succinic acid is produced. The nearer the pH is to 3.0, the more delayed is the onset of the malo-lactic fermentation. As well as the conversion of malic to lactic acid, this fermentation also sees the production of quinic and shikimic acids both of which are essential for a good flavour balance.

It will be appreciated that a number of variations in detail are possible with an improved process for the manufacture of alcoholic beverages according to the invention. For example, in the case of wine products, Shiraz grapes may be substituted by Merlot grapes, Cabernet Sauvignon grapes, Pinotage grapes, Chardonnay grapes, Chenin blanc grapes, Semillon grapes or a blend of the above mentioned grape cultivars. Also, *Aspalathus linearis* may be substituted by or mixed with *Cyclopia* in various proportions in order to further augment aroma, or to impart a mixed aroma profile. White wine cultivars can be substituted with the mentioned cultivars in order to produce a white wine product by using similar recipes and ingredients of the invention. The aforementioned variations in detail are considered as falling wholly within the scope of the present disclosure.

## Claims

1. An improved process for the manufacture of an alcoholic beverage, including the steps of providing a sugar source, subjecting the sugar source in the presence of plant material, to at least one instance of fermentation, said plant material selected from the species *Aspalathus linearis* (rooibos), *Cyclopia* (honeybush), or a combination of these, or plant material of Athrixia phylicoides (Bush Tea), wherein said fermentation process potentiates extraction of polyphenols from the plant material, and wherein said polyphenols have preservative and unique flavour- and aroma-imparting properties.

2. The improved process for the manufacture of an alcoholic beverage as claimed in claim 1, wherein said alcoholic beverage is a wine, beer product or a hard cider product.

3. The improved process for the manufacture of an alcoholic beverage as claimed in claim 1 or claim 2, wherein the sugar source is any one or more of grape cultivars, apple cultivars, or pear cultivars; and wherein the sugar source is preferably grape cultivars selected from any suitable grape cultivar, more preferably any one or more of Merlot, Shiraz (or Syrah), Pinotage, Cabernet Sauvignon, Chardonnay, Chenin Blanc, Sauvignon Blanc, Semillon or a combination of the foregoing, and wherein the alcoholic beverage is a wine product or a hard cider product.

4. The improved process for the manufacture of an alcoholic beverage as claimed in any one of the preceding claims, wherein the improved process is for the manufacture of a low-sulphur alcoholic wine, including the steps of:
- providing the sugar source and deriving a must from the sugar source;
- subjecting the must to primary fermentation; and;
- applying known clarification, stabilisation, fining, and filtration methods to the must;
- the process of manufacture including the further step of simultaneously adding plant material selected from the species *Aspalathus linearis* (rooibos), *Cyclopia* (honeybush), or a combination of these, or plant material of *Athrixia phylicoides* (Bush Tea) during the step of primary fermentation, thereby potentiating extraction of polyphenols from the plant material, useful in imparting a unique flavour and aroma to the alcoholic beverage.

5. The improved process for the manufacture of an alcoholic beverage as claimed in claim 4, wherein the step of primary fermentation increases the efficacy of polyphenol extraction, which occurs over a temperature range of between 15 degrees Centigrade and 90 degrees Centigrade, preferably between 20 degrees Centigrade and 60 degrees Centigrade, and most preferably 20 and 30 degrees Centigrade.

6. The improved process for the manufacture of an alcoholic beverage as claimed in claim 5, having a further and/or alternative step of optionally adding plant material selected from the species *Aspalathus linearis* (rooibos), *Cyclopia* (honeybush), or a combination of these, or plant material of *Athrixia phylicoides* (Bush Tea) during a secondary fermentation step, which secondary fermentation step precedes the step of applying known methods of clarification, stabilisation, fining, and filtration to the must, in order to produce a wine product.

7. The improved process for the manufacture of an alcoholic beverage as claimed in any one of the preceding claims, wherein the plant material selected from the species *Aspalathus linearis* (rooibos), *Cyclopia* (honeybush), or a combination of these, or plant material of *Athrixia phylicoides* (Bush Tea) include parts of the plant, including the roots, stems, leaves, branches, seeds, flowers, fruit and bark, or a combination of these, and wherein the plant material is in its natural form or in a processed form.

8. The improved process for the manufacture of an alcoholic beverage as claimed in any one of the preceding claims, wherein the addition of the plant material selected from the species *Aspalathus linearis* (rooibos), *Cyclopia* (honeybush), or a combination of these, or plant material of *Athrixia phylicoides* (Bush Tea), or components of the foregoing, is a replacement to oak wood or oak wood extracts in the wine making process, alternatively complements oak wood or oak wood extracts in the wine making process.

9. The improved process for the manufacture of an alcoholic beverage as claimed in any one of the preceding claims, wherein the polyphenols includes compounds from either of the groups, flavonoids or non-flavonoids, optionally wherein (a) the flavonoids include quercetin, luteolin, orientin, iso-orientin, vitexin, iso-vitexin and aspalathin, or (b) the alcoholic beverages is a wine product and the extracted polyphenols function as a natural preservative.

## Patentansprüche

1. Verbessertes Verfahren zur Herstellung eines alkoholischen Getränks, das die folgenden Schritte beinhaltet: Bereitstellen einer Zuckerquelle, Unterziehen der Zuckerquelle wenigstens einer Fermentationsinstanz in Anwesenheit von Pflanzenmaterial, wobei das genannte Pflanzenmaterial ausgewählt ist aus den Spezies *Aspalathus linearis* (Rooibos), *Cyclopia* (Honigbusch) oder einer Kombination davon, oder Pflanzenmaterial von Athrixia phylicoides (Buschtee), wobei der genannte Fermentationsprozess die Extraktion von Polyphenolen aus dem Pflanzenmaterial verstärkt und wobei die genannten Polyphenole konservierende und eindeutige geschmack- und aromaverleihende Eigenschaften haben.

2. Verbessertes Verfahren zur Herstellung eines alkoholischen Getränks nach Anspruch 1, wobei das genannte alkoholische Getränk ein Wein- oder Bierprodukt oder ein Apfelweinprodukt ist.

3. Verbessertes Verfahren zur Herstellung eines alkoholischen Getränks nach Anspruch 1 oder Anspruch 2, wobei die Zuckerquelle eine oder mehrere aus Rebsorten, Apfelsorten oder Birnensorten ist; und wobei die Zuckerquelle vorzugsweise eine Rebsorte ist, ausgewählt aus einer geeigneten Rebsorte, bevorzugter einer oder mehreren aus Merlot, Shiraz (oder Syrah), Pinotage, Cabernet Sauvignon, Chardonnay, Chenin Blanc, Sauvignon Blanc, Semillon oder einer Kombination der Obigen, und wobei das alkoholische Getränk ein Weinprodukt oder ein Apfelweinprodukt ist.

4. Verbessertes Verfahren zur Herstellung eines alkoholischen Getränks nach einem der vorherigen Ansprüche, wobei das verbesserte Verfahren für die Herstellung eines schwefelarmen alkoholischen Weins ist, einschließlich der folgenden Schritte:
- Bereitstellen der Zuckerquelle und Gewinnen eines Mosts von der Zuckerquelle;
- Unterziehen des Mosts einer primären Fermentation; und
- Anwenden von bekannten Klärungs-, Stabilisierungs-, Verfeinerungs- und Filtrationsmethoden auf den Most;
- wobei das Herstellungsverfahren den weiteren Schritt des gleichzeitigen Zugebens von Pflanzenmaterial, ausgewählt aus den Spezies *Aspalathus linearis* (Rooibos), *Cyclopia* (Honigbusch) oder einer Kombination davon, oder eines Pflanzenmaterials von *Athrixia phylicoides* (Buschtee), während des primären Fermentationsschritts beinhaltet, um dadurch die Extraktion von Polyphenolen aus dem Pflanzenmaterial zu verstärken, nützlich, um dem alkoholischen Getränk ein(en) eindeutigen/s Geschmack und Aroma zu verleihen.

5. Verbessertes Verfahren zur Herstellung eines alkoholischen Getränks nach Anspruch 4, wobei der primäre Fermentationsschritt die Wirksamkeit der Polyphenolextraktion erhöht, die über einen Temperaturbereich zwischen 15 Grad Celsius und 90 Grad Celsius, vorzugsweise zwischen 20 Grad Celsius und 60 Grad Celsius und am meisten bevorzugt zwischen 20 und 30 Grad Celsius erfolgt.

6. Verbessertes Verfahren zur Herstellung eines alkoholischen Getränks nach Anspruch 5 mit einem weiteren und/oder alternativen Schritt des optionalen Zugebens von Pflanzenmaterial, ausgewählt aus den Spezies *Aspalathus linearis* (Rooibos), *Cyclopia* (Honigbusch) oder einer Kombination davon, oder Pflanzenmaterial von *Athrixia phylicoides* (Buschtee), während eines sekundären Fermentationsschritts, wobei der sekundäre Fermentationsschritt dem Schritt des Anwendens bekannter Verfahren des Klärens, Stabilisierens, Verfeinerns und Filtrierens auf den Most vorangeht, um ein Weinprodukt zu erzeugen.

7. Verbessertes Verfahren zur Herstellung eines alkoholischen Getränks nach einem der vorherigen Ansprüche, wobei das Pflanzenmaterial ausgewählt aus den Spezies *Aspalathus linearis* (Rooibos), *Cyclopia* (Honigbusch) oder einer Kombination davon, oder Pflanzenmaterial von *Athrixia phylicoides* (Buschtee), Teile der Pflanze beinhaltet, einschließlich der Wurzeln, Stiele, Blätter, Zweige, Samen, Blüten, Früchte und Rinde, oder eine Kombination davon, und wobei das Pflanzenmaterial in seiner natürlichen Form oder in einer verarbeiteten Form vorliegt.

8. Verbessertes Verfahren zur Herstellung eines alkoholischen Getränks nach einem der vorherigen Ansprüche, wobei das Zugeben des Pflanzenmaterials, ausgewählt aus den Spezies *Aspalathus linearis* (Rooibos), *Cyclopia* (Honigbusch) oder einer Kombination davon, oder des Pflanzenmaterials von *Athrixia phylicoides* (Buschtee) oder Komponenten der Obigen, ein Ersatz für Eichenholz oder Eichenholzextrakte im Weinherstellungsverfahren, alternativ eine Ergänzung für Eichenholz oder Eichenholzextrakte im Weinherstellungsprozess ist.

9. Verbessertes Verfahren zur Herstellung eines alkoholischen Getränks nach einem der vorherigen Ansprüche, wobei die Polyphenole Verbindungen der Gruppen der Flavonoide oder Nicht-Flavonoide beinhaltet, wobei optional (a) die Flavonoide Quercetin, Luteolin, Orientin, Isoorientin, Vitexin, Iso-Vitexin und Aspalathin beinhalten, oder (b) die alkoholischen Getränke ein Weinprodukt sind und die extrahierten Polyphenole als natürliches Konservierungsmittel fungieren.

## Revendications

1. Procédé amélioré pour la fabrication d'une boisson alcoolique, incluant les étapes consistant à fournir une source de sucre, soumettre la source de sucre en présence d'un matériau végétal, à au moins un cycle de fermentation, ledit matériau végétal sélectionné parmi les espèces *Aspalathus linearis* (rooibos), *Cyclopia* (honeybush), ou une combinaison de celles-ci, ou d'un matériau végétal d'*Athrixia phylicoides* (Bush Tea), dans lequel ledit procédé de fermentation potentialise l'extraction des polyphénols à partir du matériau végétal, et dans lequel lesdits polyphénols ont des propriétés de conservation et de transmission de parfums et d'arômes uniques.

2. Procédé amélioré pour la fabrication d'une boisson alcoolique selon la revendication 1, dans lequel ladite boisson alcoolique est un vin, un produit de bière ou un produit de cidre.

3. Procédé amélioré pour la fabrication d'une boisson alcoolique selon la revendication 1 ou la revendication 2, dans lequel la source de sucre est un quelconque ou plusieurs cultivars parmi des cultivars de raisins, des cultivars de pommes, ou des cultivars de poires ; et dans lequel la source de sucre est préférablement des cultivars de raisins sélectionnés parmi tout cultivar de raisin adapté, plus préférablement un quelconque ou plusieurs cultivars parmi le Merlot, le Shiraz (ou Syrah), le Pinotage, le Cabernet Sauvignon, le Chardonnay, le Chenin blanc, le Sauvignon blanc, le Sémillon ou une combinaison de ce qui précède, et dans lequel la boisson alcoolique est un produit de vin ou un produit de cidre.

4. Procédé amélioré pour la fabrication d'une boisson alcoolique selon l'une quelconque des revendications précédentes, dans lequel le procédé amélioré est pour la fabrication d'un vin alcoolique à faible teneur en soufre, incluant les étapes consistant à :
- fournir la source de sucre et tirer un moût de la source de sucre ;
- soumettre le moût à une fermentation primaire ; et
- appliquer des procédés connus de clarification, de stabilisation, de collage, et de filtration au moût ;
- le procédé de fabrication incluant l'étape supplémentaire consistant à ajouter simultanément du matériau végétal sélectionné parmi les espèces *Aspalathus linearis* (rooibos), *Cyclopia* (honeybush), ou une combinaison de celles-ci, ou un matériau végétal *d'Athrixia phylicoides* (Bush Tea) pendant l'étape de fermentation primaire, potentialisant ainsi l'extraction des polyphénols à partir du matériau végétal, utiles dans la transmission de parfums et d'arômes uniques à la boisson alcoolique.

5. Procédé amélioré pour la fabrication d'une boisson alcoolique selon la revendication 4, dans lequel l'étape de fermentation primaire augmente l'efficacité de l'extraction des polyphénols, qui a lieu dans une plage de températures situées entre 15 degrés centigrades et 90 degrés centigrades, préférablement situées entre 20 degrés centigrades et 60 degrés centigrades, et de manière préférée entre toutes 20 et 30 degrés centigrades.

6. Procédé amélioré pour la fabrication d'une boisson alcoolique selon la revendication 5, ayant une étape supplémentaire et/ou autre consistant à ajouter simultanément du matériau végétal sélectionné parmi les espèces *Aspalathus linearis* (rooibos), *Cyclopia* (honeybush), ou une combinaison de celles-ci, ou du matériau végétal d'*Athrixia phylicoides* (Bush Tea) pendant une étape de fermentation secondaire, laquelle étape de fermentation secondaire précède l'étape consistant à appliquer des procédés connus de clarification, de stabilisation, de collage, et de filtration au moût, afin de produire un produit de vin.

7. Procédé amélioré pour la fabrication d'une boisson alcoolique selon l'une quelconque des revendications précédentes, dans lequel le matériau végétal sélectionné parmi les espèces *Aspalathus linearis* (rooibos), *Cyclopia* (honeybush), ou une combinaison de celles-ci, ou le matériau végétal d'*Athrixia phylicoides* (Bush Tea) inclut des parties de la plante, incluant les racines, les tiges, les feuilles, les branches, les graines, les fleurs, les fruits et l'écorce, ou une combinaison de ceux-ci, et dans lequel le matériau végétal est sous sa forme naturelle ou sous une forme transformée.

8. Procédé amélioré pour la fabrication d'une boisson alcoolique selon l'une quelconque des revendications précédentes, dans lequel l'ajout du matériau végétal sélectionné parmi les espèces *Aspalathus linearis* (rooibos), *Cyclopia* (honeybush), ou une combinaison de celles-ci, ou du matériau végétal d'*Athrixia phylicoides* (Bush Tea), ou de composants des éléments précédents, est un remplacement du bois de chêne ou des extraits de bois de chêne dans le procédé de fabrication du vin, en variante complète le bois de chêne ou les extraits de bois de chêne dans le procédé de fabrication du vin.

9. Procédé amélioré pour la fabrication d'une boisson alcoolique selon l'une quelconque des revendications précédentes, dans lequel les polyphénols incluent des composés de l'un ou l'autre des groupes des flavonoïdes ou des non flavonoïdes, dans lequel facultativement (a) les flavonoïdes incluent la quercétine, la lutéoline, l'orientine, l'isoorientine, la vitexine, l'isovitexine et l'aspalathine, ou (b) la boisson alcoolique est un produit de vin et les polyphénols extraits fonctionnent comme un conservateur naturel.
